(19) Europäisches Patentamt
European Patent Office
Office européen des brevets

(11) Publication number: 0 188 535
B1

(12) # EUROPEAN PATENT SPECIFICATION

(45) Date of publication of patent specification: 16.08.90

(51) Int. Cl.[5]: C 07 C 67/08

(21) Application number: 85903567.7

(22) Date of filing: 28.06.85

(86) International application number:
PCT/US85/01230

(87) International publication number:
WO 86/00300 16.01.86 Gazette 86/02

(54) **HIGH MOLECULAR WEIGHT LIQUID ESTERS.**

(30) Priority: 28.06.84 US 625487

(43) Date of publication of application:
30.07.86 Bulletin 86/31

(45) Publication of the grant of the patent:
16.08.90 Bulletin 90/33

(84) Designated Contracting States:
AT BE CH DE FR GB IT LI NL SE

(56) References cited:
US-A-3 769 215
US-A-4 165 303

No relevant documents have been disclosed

(73) Proprietor: HENKEL CORPORATION (a Delaware corp.)
7900 West 78th Street
Minneapolis Minnesota 55435 (US)

(72) Inventor: LEENDERS, Peter
5317 Hollywood Road
Edina, MN 55436 (US)

(74) Representative: von Kreisler, Alek, Dipl.-Chem. et al
Deichmannhaus am Hauptbahnhof
D-5000 Köln 1 (DE)

EP 0 188 535 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

1. Field of the invention

High molecular weight liquid esters are provided which are usfeul as lubricants, release agents, plasticizers, solvents or a modifier additive for such compositions. Advantageously they demonstrate characteristics, such as fluidity and high molecular weight combined with lubricating, plasticizing, solvent and mold releasing properties.

2. Statement of related art

Processes have been developed to produce lubricants by the esterification of alcohols and acids. For example, *U.S. Patent No. 2,862,013* relates to the esterification of branched chain liquid alcohols containing from 24 to 28 carbon atoms with acids having from 12 to 18 carbon atoms to produce functional fluids on the low end of the range up to high melting point waxes on the high end of the range.

Another reference which discusses the production of lubricants is *U.S. Patent No. 4,157,990*. This reference indicates that esters of polycarboxylic acids and long chain aliphatic monovalent alcohols can be used to give fluidity to thermoplastic materials. This reference further describes the esterification of dicarboxylic acids, and monocarboxylic acids having from 12 to 30 carbon atoms with aliphatic polyols to produce lubricant takifiers. This reference also indicates that the esters of long chained fatty acids are used to improve fluidity.

It has now been discovered that the esterification of branched, unsaturated guerbet alcohols having a minimum of 28 carbon atoms with polymeric fatty acids having a minimum of 24 carbon atoms will produce liquid lubricants having an unusually high molecular weight combined with other desired characteristics such as low solidification points, low mobility, vapor pressure and volatility. These properties make the liquid, high molecular weight esters of the instant invention useful as lubricants, plasticizers, and mold release agents. The usefulness of these esters, moreover, is enhanced by the high molecular weight of the constituents (the original polymeric fatty acids and alcohols).

Brief description of the invention

The composition provided for comprises: A liquid ester of a polymeric fatty acid and a branched aliphatic primary guerbet alcohol, the alcohol having from 28 to 60 carbon atoms.

The polymeric fatty acids employed in this invention are well known and commercially available. They may be fractionated to provide polymeric fatty acids with high dimer or trimer content and can be hydrogenated to be substantially saturated, although unsaturation is preferred. Those employed herein will generally have from 24 to 90 carbon atoms.

The branched aliphatic primary guerbet alcohols used in this invention are prepared by the condensation (guerbet reaction) of alcohols containing from 14 to 30 carbon atoms which will provide the branched aliphatic primary guerbet alcohols having the 28—60 carbon atoms.

The liquid esters of this invention which will generally contain 52—150 carbon atoms can also be represented by the formula:

$$R_1-O-\overset{\overset{\displaystyle O}{\|}}{C}-R_2-(\overset{\overset{\displaystyle O}{\|}}{C}-O-R_3)_n$$

where $R_1$ is the branched aliphatic hydrocarbon radical or ligand of the primary branched guerbet alcohol, and, as such, has a minimum of 28 carbon atoms; $R_2$ is the hydrocarbon radical or ligand of a polymeric fatty acid of a monomeric fatty acid containing 12 to 24 carbon atoms; n is an integer which depends on the number of carboxyl groups present in the polymeric form of the polymeric fatty acid and $R_3$ is hydrogen or the branched aliphatic hydrocarbon radical or ligand of the primary, branched guerbet alcohol, and has a minimum of 28 carbon atoms. $R_3$ and $R_1$ can be from the same or different alcohols. The range of carbon atoms for $R_2$ is generally from 22 to 88.

The esters of the instant invention advantageously are capable of retaining desirable properties in spite of the breakdown typical to esters particularly when exposed to high temperature conditions in their area of use. This breakdown results in significant amounts of the original ester forming acids and alcohols being present either during use or storage. The instant invention prolongs life and usefulness of the esters in spite of these quantities of acids and alcohols due to the molecular weight, character, and properties of the alcohols and acids used as feeds. The esters described herein, thus, in spite of typical breakdown, tend to retain such properties as high vapor pressure, good fluidity, low volatility, high smoke points, low flammability, good solvent properties, and good lubrication properties.

Detailed description of the invention

The present invention is related to a liquid ester containing 52 to 150 carbon atoms having the formula:

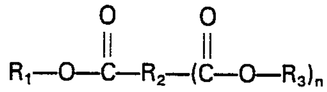

where $R^1$ is the branched aliphatic hydrocarbon radical of a primary branched guerbet alcohol having from 28 to 60 carbon atoms, $R_2$ is the hydrocarbon radical of a polymeric fatty acid of a monomeric fatty acid containing 12 to 24 carbon atoms, $R_3$ is hydrogen or a branched aliphatic hydrocarbon radical of a primary branched guerbet alcohol having from 28 to 60 carbon atoms and n is an integer of from 1 to 5.

$R_3$ and $R_1$ can be from the same or different alcohols. The range of carbon atoms for $R_2$ is generally from 22 to 88. The integer n is preferably 1 or 2 for the respective dimers and trimers which contain 2 and 3 carboxylic groups respectively. When n is 3, 4 or 5, the higher polymeric forms, such as the tetramer, pentamer or hexamer, are included. These are generally found in small amounts, if any, in available polymeric fatty acids.

The guerbet alcohols used for esterification should have at least one branch per molecule. This branch, moreover, shold not be off of the hydroxyl carbon, thus, the guerbet alcohols must also be primary, with from 28 to 60 carbon atoms. A mixture of branched aliphatic primary guerbet alcohols can be used in esterification. Preferably the guerbet alcohol feed is unsaturated, the unsaturation level of the feed acceptably, having a minimum iodine value of 40; most preferably, the iodine value being in the range of from 70 to 120.

Guerbet alcohols of the type called for by the instant process can be prepared from other alcohols by condensation or "guerbetization". A preferred method to obtain the guerbet alcohols required by the instant invention is to use a guerbet type reaction whereby oleyl alcohols are condensed to form branched dioleyl primary alcohols. This type of reaction is well-known in the art, and can be found described in Swiss Patent 231,252 to Henkel and Cie GmbH, and in U.S. Patent Nos. 2,836,628; 3,119,880; 3,328,470; 3,558,716; 4,011,273; and 3,479,412. Any of these described methods can be used to condense oleylic alcohols forming primary branched unsaturated guerbet alcohols and are suitable to produce the alcohols used in the instant invention as would be any method capable of condensing an oleylic alcohol without destroying unsaturation. The instant invention, however, is not limited to guerbet alcohols produced by these methods, since any primary, branched alcohols having from 28 to 60 carbon atoms can be used including any that are naturally found.

Both branched or linear primary guerbet alcohols can be used in condensation or guerbet-type reactions to produce the guerbet alcohols needed in the instant invention. The guerbet alcohols used in such condensation type reactions must have a minimum of 14 carbon atoms, an acceptable range being from 14 to 30 carbon atoms. These will thus provide primary branched guerbet alcohols having 28—60 carbon atoms. Preferably, the esterification feed is produced from guerbet alcohols having from 14 to 24 carbon atoms, and are most preferably derived from natural fats and oils. One such preferred guerbet alcohol is oleyl alcohol.

The acids employed to form the esters of the instant invention are polymeric fatty acids, generally having from 24 to 90 carbon atoms. Preferably, those used are the commercially available acids prepared from $C_{18}$ fatty acids and have from 36 to 54 carbon atoms in their respective dimer and trimer forms. Both saturated and unsaturated acids are used although more preferably the acids are unsaturated.

The polymeric fatty acids are well known and readily available commercially. One method of preparing such products is shown in U.S. Patent 3,157,681. This patent also contains a general description of various other methods such as U.S. Patent 2,347,562; 2,417,738; 2,426,489; 2,450,332; 2,673,184; 2,793,219; 2,793,220 and 2,955,121, which description is hereby incorporated by reference. Briefly the preferred method of preparing polymeric fatty acids is by a process of polymerization consisting of heating unsaturated fatty acids (either an individual acid or mixtures thereof) derived from fats or oils at temperatures in the range of 180—200°C. in the presence of a clay catalyst. The usual temperature employed is 200—250°C with 230°C being preferred.

The term "polymeric fatty acids" as used herein is intended to be generic to polymerized acids obtained from "fatty acids". The term "fatty acids" is intended to include saturated, ethylenically unsaturated and acetylenically unsaturated, naturally occurring and synthetic, monobasic aliphatic acids containing from 12 to 24 carbon atoms, which will provide polymeric fatty acids containing from 24 to 90 carbon atoms in their predominant dimer and trimer forms.

The saturated, ethylenically unsaturated and acetylenically unsaturated fatty acids are generally polymerized by somewhat different techniques, but because of the functional similarity of the polymerization products, they all are generally referred to as "polymeric fatty acids".

Saturated fatty acids are difficult to polymerize but polymerization can be obtained at elevated temperatures with a peroxidic catalyst such as di-t-butyl peroxide. Because of the generally low yields of polymeric products, these materials are not currently commercially significant. Suitable saturated fatty acids include branched and straight chain acids such as caprylic acid, pelargonic acid, capric acid, lauric acid, myristic acid, palmitic acid, isopalmitic acid, stearic acid, arachidic acid, behenic acid, and lignoceric acid.

The ethylenically unsaturated acids are much more readily polymerized. Catalytic or non-catalytic polymerization techniques can be employed. The non-catalytic polymerization generally requires a higher

temperature. Suitable catalysts for the polymerization includes acid or alkaline clays, di-t-butyl peroxide, boron trifluoride and other Lewis acids, anthraquinone, sulfur dioxide and the like. Suitable monomers include the branched and straight chain, poly- and mono-ethylenically unsaturated acids such as 3-octenoic acid, 11-dodecanoic acid, lineric acid, lauroleic acid, myristoleic acid, tsuzuic acid, palmitoleic acid, gadoleic acid, cetoleic acid, nervonic acid, moroctic acid, timnodonic acid, eicosatetraenoic acid, nisinic acid, scoliodonic acid and chaulmoogric acid.

The acetylenically unsaturated fatty acids can be polymerized by simply heating the acids. Polymerization of these highly reactive materials will occur in the absence of a catalyst. The acetylenically unsaturated acids occur only rarely in nature and are expensive to synthesize. Therefore, they are not currently of commercial significance. Any acetylenically unsaturated fatty acid, both straight chain and branched chain, both mono-unsaturated and poly-unsaturated, are useful monomers for the preparation of the polymeric fatty acids. Suitable examples of such materials include 10-undecynoic acid, tariric acid, stearolic acid, behenolic acid and isamic acid.

Because of their ready availability and relative ease of polymerization, oleic and linoleic acid are the preferred starting materials for the preparation of the polymeric fatty acids, a mixture of which is found in tall oil fatty acids, which is accordingly the most common commercial starting material.

Having obtained the polymeric fatty acids or derivatives as described above, they may then be fractionated, for example, by conventional techniques of distillation or solvent extraction. Where color and stability of the polymers are particularly important, hydrogenated and fractionated polymeric fatty acids are the preferred starting materials.

Typical compositions of commercially available polymeric fatty acids, based on unsaturated $C_{18}$ fatty acids, which are suitable for the esters of the instant invention are:

$C_{18}$ monobasic acids ("monomer") 5—15% by weight;
$C_{36}$ dibasic acid ("dimer") 60—80% by weight;
$C_{54}$ (and higher) ("trimer") polybasic acids 10—35% by weight.

The relative ratios of monomer, dimer and trimer (or higher) in unfractionated polymeric fatty acids are dependent on the nature of the starting material and the conditions of polymerization. For the purposes of this invention, the term monomeric fatty acids refers to the unpolymerized monomeric acids; the term dimeric fatty acids refers to the dimeric acids or derivatives (formed by the dimerization of two fatty acid molecules); and the term trimeric fatty acids refers to the residual higher polymeric forms consisting primarily of trimeric acids or derivatives, but containing some higher polymeric forms.

Reference has been made above to the monomeric dimeric and trimeric fatty acids present in the polymeric fatty acids. The amounts of monomeric fatty acids, often referred to as monomer (M), dimeric fatty acids, often referred to as dimer (D), and trimeric or higher polymeric forms, often referred to as trimer (T), may be determined by gas liquid chromatography (GLC) of the methyl esters of the polymeric fatty acids. In this method of analysis, an intermediate (I) is seen between the monomer and dimer forms. It is desirable that this intermediate be low, but polymeric fatty acids generally having less than 10%, and preferably less than 6% intermediate by weight are satisfactory. Unless otherwise indicated, this analytical method was employed in the analysis of the polymeric fatty acids employed in this invention.

Mixtures may be fractionated by suitable means such as high vacuum distillation or solvent extraction techniques so as to obtain dimer acid cuts of greater than 80%, and above 90% or 95% dimeric species by weight. These dimer-rich fractions can also be used as the starting materials in an esterification to produce the liquid esters of the instant invention. Mixtures of polymeric fatty acids dimeric and trimeric that can also be used to produce these esters.

The liquid esters of the instant invention are obtained by esterification procedures. Any known esterification process is acceptable. For example, an esterification which will produce the esters of the instant invention can be done by the combination of the previously described guerbet alcohols and polymeric fatty acids at temperatures in the range of from about 50° to about 300°C.

Generally, the reaction will proceed without catalysts although there are a variety of esterification catalysts, and any of these may be used to produce the compounds of the instant invention. Representative, but non-exhaustive examples of such catalysts are: mineral acids, or bases such as sodium hydroxide and potassium hydroxide, alkali metal alkoxides such as sodium or potassium alkoxide, aluminum alkoxide, and divalent metal salts such as zinc salts, and manganese acetate, sodium methylate, and metal such as zinc and nickel. Acidic resins have also been used as esterification catalysts. An example of such catalysts which are also acceptable are the sulfonated polystyrene resins. Other substances which have been used to catalyze esterification reactions which may be used to produce the esters of the instant invention are: phenol, formaldehyde, tin oxides and hydrosulfonic acid resins. To produce the esters of the instant invention preferably the temperature is from 75°C to about 300°C.

Pressure is not a critical factor and any pressure convenient for the esterification process used is acceptable. It may, however, be preferred to produce the esters herein described by an esterification process conducted under less than atmospheric pressure or under an inert atmosphere. This would decrease oxidation and ensure a clear product which would be acceptable for use as a plasticizer.

The esterification reaction can be controlled so that the acidity of the product is higher or lower. Thus,

for example, it is possible to conduct step-wise esterifications which substantially completes the esterification of the dibasic acids, but which would esterify two of the acid ligands of the high polymeric fatty acids. For such an esterification the alcohol to polymeric fatty acid mole ratio should be at least 2 to 1, preferably 2.25 to 1. This would produce a less acidic ester product than would a process having only that amount of guerbet alcohol necessary to esterify one acid ligand per polymeric fatty acid molecule. To esterify only one ligand there should be at least a sufficient amount of alcohol to ensure an esterification product of such a highly acidic nature, therefore, the guerbet alcohol to polymeric fatty acid mole ratio should be at least 1 to 1; and more preferably 1.5 to 1. For more complete esterifications, the guerbet alcohol concentration should be increased. For a complete esterification the ratio of guerbet alcohol to acid ligand should be 1:1. An acceptable overall range for the mole ratio of guerbet alcohol to polymeric fatty acid is from 1 to 1 to 4 to 1.

The following examples are offered to illustrate the instant invention and not to limit it. All parts and percentages are by weight unless otherwise specified.

Example I

Starting polymeric fatty acid materials used to prepare the esters described herein were a predominantly trimeric fatty acid (commercial designation Versatryme® 213) and a predominantly dimeric fatty acid (commercial designation Versadyme® 52). The composition of these acids, which are polymerized tall oil acids determined by gas liquid chromatography (GLC) of the methyl esters of the polymeric fatty acids, as earlier noted, are reported as the following typical composition according to the manufacturers specification:

| | | 213 Product | 52 Product |
|---|---|---|---|
| % monomer (M) | by weight | 4% max | 2% max |
| % dimer (D) | by weight | 25—45% | 88% min |
| % trimer (T) | by weight | 50—75% | 5% max |
| % intermediate (I) | by weight | 3% max | 6% max |

The branched unsaturated primary guerbet alcohols used were essentially dioleyl guerbet alcohol (alcohol A) and having from 32 to 38 carbon atoms per molecule, with less than 10% trioleyl guerbet alcohols. The iodine value of these alcohols was 90 to 95. Primary saturated, branched guerbet alcohols were also esterified.

Three esterifications were completed, one using saturated branched guerbet alcohols having molecular weight ranges of from 24 to 28 carbon atoms (alcohol B) and two with alcohols having from 32 to 36 carbon atoms (alcohol C).

In all of the esterifications, the guerbet alcohol to acid ligand ratio used was 1:1. Thus, equal equivalents of acid and guerbet alcohol were mixed together with 0.25% by weight tin oxide. The following general procedure was used: The reaction mixture was heated at 185°C until the evolution of water was completed, while a nitrogen sparge was applied. To help complete the reaction further, vacuum was applied down to 13332.2 Pa (100 torr) and the solution was again heated to 200°C until water formation again stopped. Thereafter, a vacuum of 666.6 Pa (5 torr) or less was applied and the mixture was heated to the range of about 260° to 280°C but no further water formation was observed. The reaction mixture was then cooled to 95°C and filtered. The liquids were clear but slightly colored. All esters were fluid, and the esters of the trimer acids were substantially less viscous than the acid itself.

Esters of dimer and trimer acids

| The ester of | | | | | |
|---|---|---|---|---|---|
| Alcohol | Polymeric acid | Product acid value | Product OH value | Sapon. value | Fluid at |
| A | 213 | 5.7 | 0.11 | 70.0 | fluid at −8°C |
| A | 52 | 4.0 | 4.5 | 70.1 | fluid at −8°C |
| B | 213 | 2.4 | 5.7 | 81.8 | fluid at 4°C<br>solid at −8°C |
| C | 213 | 12.2 | 0.4 | 87.3 | solid at −8°C |
| C | 52 | 17.3 | 8.4 | | solid at −8°C |

## Claims

1. A liquid ester containing 52 to 150 carbon atoms having the formula:

$$R_1-O-\overset{\overset{O}{\|}}{C}-R_2-(\overset{\overset{O}{\|}}{C}-O-R_3)_n$$

where $R^1$ is the branched aliphatic hydrocarbon radical of a primary branched guerbet alcohol having from 28 to 60 carbon atoms, $R_2$ is the hydrocarbon radical of a polymeric fatty acid of a monomeric fatty acid containing 12 to 24 carbon atoms, $R_3$ is hydrogen or a branched aliphatic hydrocarbon radical of a primary branched guerbet alcohol having from 28 to 60 carbon atoms and n is an integer of from 1 to 5.

2. An ester as described in claim 1 wherein the iodine value of the alcohol is in the range of from 70 to 120.

3. An ester as defined in claim 1 in which said monomeric fatty acid is an unsaturated monocarboxylic fatty acid containing 18 carbon atoms.

4. An ester as defined in claim 1 in which said unsaturated fatty acid is oleic acid.

5. An ester as defined in claim 1 in which said unsaturated fatty acid is linoleic acid.

6. An ester as defined in claim 1 in which said polymeric fatty acid is polymerized tall oil fatty acid.

7. An ester as defined in claim 1 wherein $R_2$ is a hydrocarbon radical of a polymerized tall oil fatty acid and n is 2.

8. An ester as defined in claim 1 wherein $R_2$ is a hydrocarbon radical of a polymerized tall oil fatty acid and n is 3.

9. An ester as defined in claim 1 wherein $R_1$ and $R_3$ are the same and are the hydrocarbon residue of an oleyl guerbet alcohol.

10. A lubricant composition containing the liquid ester of claim 1.

## Patentansprüche

1. Flüssiger Ester, enthaltend 52 bis 150 Kohlenstoff-Atome, mit der Formel

$$R_1-O-\overset{\overset{O}{\|}}{C}-R_2-(\overset{\overset{O}{\|}}{C}-O-R_3)_n$$

in der

$R_1$ der verzweigte aliphatische Kohlenwasserstoff-Rest eines primären verzweigten Guerbet-Alkohols mit 28 bis 60 Kohlenstoff-Atomen ist,

$R_2$ der Kohlenwasserstoff-Rest einer polymeren Fettsäure einer monomeren Fettsäure mit 12 bis 24 Kohlenstoff-Atomen ist,

$R_3$ Wasserstoff oder ein verzweigter aliphatischer Kohlenwasserstoff-Rest eines primären verzweigten Guerbet-Alkohols mit 28 bis 60 Kohlenstoff-Atomen ist und

n eine ganze Zahl von 1 bis 5 ist.

2. Ester nach Anspruch 1, worin die lod-Zahl des Alkohols im Bereich von 70 bis 120 liegt.

3. Ester nach Anspruch 1, worin die monomere Fettsäure eine ungesättigte Monocarboxyl-Fettsäure mit 18 Kohlenstoff-Atomen ist.

4. Ester nach Anspruch 1, worin die ungesättigte Fettsäure Ölsäure ist.

5. Ester nach Anspruch 1, worin die ungesättigte Fettsäure Linolsäure ist.

6. Ester nach Anspruch 1, worin die polymere Fettsäure polymerisierte Tallöl-Fettsäure ist.

7. Ester nach Anspruch 1, worin $R_2$ ein Kohlenwasserstoff-Rest einer polymerisierten Tallöl-Fettsäure ist und n 2 ist.

8. Ester nach Anspruch 1, worin $R_2$ ein Kohlenwasserstoff-Rest einer polymerisierten Tallöl-Fettsäure ist und n 3 ist.

9. Ester nach Anspruch 1, worin $R_1$ und $R_3$ gleich sind und die Kohlenwasserstoff-Reste eines Oleyl-Guerbet-Alkohols sind.

10. Gleitmittel-Zusammensetzung, enthaltend den flüssigen Ester nach Anspruch 1.

## Revendications

1. Ester liquide contenant 52 à 150 atomes de carbone et présentant la formule suivante:

$$R_1-O-\overset{\overset{O}{\|}}{C}-R_2-(\overset{\overset{O}{\|}}{C}-O-R_3)_n$$

dans laquelle $R_1$ est le radical hydrocarboné aliphatique ramifié d'un alcool de guerbet primaire ramifié ayant de 28 à 60 atomes de carbone, $R_2$ est le radical hydrocarboné d'un acide gras polymère d'un acide gras monomère contenant 12 à 24 atomes de carbone, $R_3$ est un atome d'hydrogène ou un radical hydrocarboné aliphatique ramifié d'un alcool de guerbet primaire ramifié ayant de 28 à 60 atomes de carbone et n est un nombre entier de 1 à 5.

2. Ester selon la revendication 1 dans lequel l'indice d'iode de l'alcool est compris dans la gamme de 70 à 120.

3. Ester selon la revendication 1 dans lequel ledit acide gras monomère est un acide gras monocarboxylique insaturé contenant 18 atomes de carbone.

4. Ester selon la revendication 1 dans lequel ledit acide gras insaturé est l'acide oléique.

5. Ester selon la revendication 1 dans lequel ledit acide gras insaturé est l'acide linoléique.

6. Ester selon la revendication 1 dans lequel ledit acide gras polymère est un acide gras de tallol polymérisé.

7. Ester selon la revendication 1 dans lequel $R_2$ est un radical hydrocarboné d'un acide gras de tallol polymérisé et n est égal à 2.

8. Ester selon la revendication 1 dans lequel $R_2$ est un radical hydrocarboné d'un acide gras de tallol polymérisé et n est égal à 3.

9. Ester selon la revendication 1 dans lequel $R_1$ et $R_3$ sont identiques et sont le résidu hydrocarboné d'un alcool de guerbet oléique.

10. Composition lubrifiante contenant l'ester liquide selon la revendication 1.